# EUROPÄISCHE PATENTANMELDUNG

(11) **EP 1 576 995 A2**
(43) Veröffentlichungstag der Anmeldung: **21.09.2005**
(21) Anmeldenummer: 05004754.7
(22) Anmeldetag: 04.03.2005
(51) Int. Cl.: B01D 3/14, B01D 3/32

(54) **Verfahren zur kontinuierlichen Zerlegung von Stoffgemischen**

(30) Priorität: 17.03.2004 DE 102004012904
(71) Anmelder: LANXESS Deutschland GmbH, 51369 Leverkusen (DE)
(72) Erfinder: Müller, Dirk, Dr., 51467 Bergisch Gladbach (DE); Wagner, Paul, Dr., 40597 Düsseldorf (DE); Lindel, Hans, Dr., 51373 Leverkusen (DE); Langer, Reinhard, Dr., 47918 Tönisvorst (DE)

(57) **Zusammenfassung**

Die vorliegende Erfindung betrifft ein Verfahren zur kontinuierlichen, destillativen Zerlegung von Stoffgemischen in zumindest drei Fraktionen unter Verwendung einer Destillationsvorrichtung bestehend zumindest aus einem Dünnschichtverdampfer (11) und einer Kolonne (2).

## Beschreibung

Die vorliegende Erfindung betrifft ein Verfahren zur kontinuierlichen, destillativen Zerlegung von Stoffgemischen in zumindest drei Fraktionen unter Verwendung einer Destillationsvorrichtung bestehend zumindest aus einem Dünnschichtverdampfer und einer Kolonne.

Destillationsverfahren zur kontinuierlichen Zerlegung von Stoffgemischen in drei Fraktionen sind prinzipiell bekannt und beispielsweise zusammenfassend beschrieben in Stichlmair, Fair, Distillation, Wiley-VCH, 1998.

Üblicherweise werden dazu zwei Destillationsschritte nacheinander ausgeführt, um zunächst eine erste Fraktion abzutrennen und dann das verbleibende Stoffgemisch in zwei weitere Fraktionen zu zerlegen (siehe zum Beispiel DE-OS 197 33 903, DE-OS 100 20 766, DE-OS 29 28 553, EP-A 555 335 und US 4,935,555). Alternativ kann aber auch einstufig eine Zerlegung in drei Fraktionen erfolgen, indem ein Seitenstrom aus einer Destillationskolonne entnommen wird (siehe zum Beispiel DE-OS 42 14 738).

Nachteilig an den bislang bekannten Verfahren ist die Tatsache, dass die Zerlegung von Stoffgemischen mit thermisch empfindlichen Komponenten nur in unzulänglicher Weise möglich ist.

Unter thermisch empfindlichen Komponenten sind im Zusammenhang dieser Erfindung Komponenten zu verstehen, die sich unter thermischer Belastung, wie sie typischerweise unter Destillationsbedingungen auftreten, mit zunehmender Verweildauer zur Zersetzung neigen, wodurch unerwünschte Ausbeuteverluste und/oder Qualitätsbeeinträchtigungen wie geringe Reinheit oder hohe Farbzahlen des Wertproduktes entstehen.

Aus DD-OS 229 397 ist bekannt, einen Dünnschichtverdampfer mit einer Kolonne zu verbinden, um gleichzeitig eine gute Trennwirkung und einen schonenden Energieeintrag zu erzielen. Hierzu werden Dünnschichtverdampfer und Kolonne so gekoppelt, dass der im Dünnschichtverdampfer erzeugte Dampf in die Kolonne geführt wird, die am unteren Ende der Kolonne anfallende Fraktion entnommen und dem Dünnschichtverdampfer wieder zugeführt wird. Parallel dazu wird der Kolonne ein dampfförmiger Seitenstrom entnommen. Auf diese Weise kann das Zulaufgemisch in insgesamt drei Fraktionen zerlegt werden. Das beschriebene Verfahren eignet sich jedoch nicht für Stoffgemische, bei denen die Hauptkomponente in hohen Anteilen, d.h. in Gewichtsanteilen von zumindest 50 %, im Zulaufgemisch vorliegt und von der Hauptkomponente sowohl leichtersiedende als auch schwerersiedende Nebenkomponenten abgetrennt werden müssen.

Es bestand daher das Bedürfnis, ein Verfahren und eine Vorrichtung bereitzustellen, die die besonders schonende thermische Zerlegung von Stoffgemischen ermöglicht, die neben der Hauptkomponente, die einen Anteil von zumindest 50 % im Zulaufgemisch besitzt, leichter- und schwerersiedende Komponenten enthalten.

Es wurde nun ein Verfahren zur kontinuierlichen, destillativen Zerlegung von Stoffgemischen bestehend zumindest aus einer mittelsiedenden Hauptfraktion sowie mindestens einer leichtersiedenden und mindestens einer schwerersiedenden Nebenfraktion gefunden, das dadurch gekennzeichnet ist, dass
- eine Destillationsvorrichtung, bestehend aus zumindest einem Dünnschichtverdampfer und einer Kolonne, kontinuierlich über die Kolonne mit einem Zulauf beaufschlagt wird und
- der Zulauf zumindest in
   a) eine leichtersiedende Nebenfraktion und
   b) eine Fraktion, die die mittelsiedende Hauptfraktion und die schwerersiedende Nebenfraktion enthält, getrennt wird, und
- die am Kolonnenfuß anfallende Fraktion, die die mittelsiedende Hauptfraktion und die schwerersiedende Nebenfraktion enthält, in flüssiger Form dem Dünnschichtverdampfer zugeführt wird und
- die Fraktion, die die mittelsiedende Hauptfraktion und die schwerersiedende Nebenfraktion enthält, im Dünnschichtverdampfer in
   a) eine schwerersiedende Nebenfraktion und
   b) einen die mittelsiedende Hauptfraktion enthaltenden Dampfstrom getrennt wird und
- der die mittelsiedende Hauptfraktion enthaltende Dampfstrom teilweise kondensiert und teilweise der Kolonne zugeführt wird.

In einer alternativen Ausführungsform des erfindungsgemäßen Verfahrens kann der Zulauf auch in den Dünnschichtverdampfer eingespeist werden. Daher ist von der Erfindung weiterhin ein Verfahren zur kontinuierlichen, destillativen Zerlegung von Stoffgemischen bestehend zumindest aus einer mittelsiedenden Hauptfraktion sowie mindestens einer leichtersiedenden und mindestens einer schwerersiedenden Nebenfraktion umfasst, das dadurch gekennzeichnet ist, dass
- eine Destillationsvorrichtung, bestehend aus zumindest einem Dünnschichtverdampfer und einer Kolonne, kontinuierlich über den Dünnschichtverdampfer mit einem Zulauf beaufschlagt wird und
- der Zulauf zumindest in
   c) einen die leichtersiedende Nebenfraktion die mittelsiedende Hauptfraktion enthaltenden Dampfstrom und
   d) die schwerersiedende Nebenfraktion getrennt wird, und
- aus dem die leichtersiedende Nebenfraktion und die mittelsiedende Hauptfraktion enthaltenden Dampfstrom die mittelsiedende Hauptfraktion zumindest teilweise kondensiert wird und der verbleibende Dampfstrom der Kolonne zugeführt wird und
- der verbleibende Dampfstrom in der Kolonne in einen die leichtersiedende Nebenfraktion enthaltenden Dampfstrom und gegebenenfalls eine am Kolonnenfuß anfallende, die mittelsiedende Hauptfraktion enthaltende Flüssigkeit getrennt wird.

Bevorzugt ist jedoch die Ausführungsform des erfindungsgemäßen Verfahrens, in dem der Zulauf über die Kolonne erfolgt.

Es sei darauf hingewiesen, dass vom Umfang der Erfindung auch beliebige Kombinationen von Merkmalen und deren Vorzugsbereichen umfasst sind.

Unter dem Begriff schwerersiedende Fraktion sind solche Stoffe oder Stoffgemische zu verstehen, die einen höheren Siedepunkt besitzen als die mittelsiedende Hauptfraktion.

Unter dem Begriff leichtersiedende Fraktion sind solche Stoffe oder Stoffgemische zu verstehen, die einen niedrigeren Siedepunkt besitzen als die mittelsiedende Hauptfraktion.

Der Begriff Hauptfraktion bezeichnet denjenigen Stoff oder dasjenige Stoffgemisch, das im Zulaufgemisch einem Anteil von mehr als 50 Gew.-%, bevorzugt mehr als 70 Gew.-% und besonders bevorzugt mehr als 85 Gew.-% aufweist. Bevorzugte Hauptfraktionen sind Einzelverbindungen und Azeotrope, bevorzugt jedoch Einzelverbindungen.

Der Begriff Nebenfraktion bezeichnet denjenigen Stoff oder diejenigen Stoffgemische, die im Zulaufgemisch einen geringeren Gewichtsanteil aufweisen als die Hauptfraktion.

Im erfindungsgemäßen Verfahren wird die Destillationsvorrichtung kontinuierlich mit einem Zulauf beaufschlagt.

Der Begriff kontinuierlich schließt zeitliche Änderungen der Zulaufmenge ebenso ein wie vorübergehende Unterbrechungen des Zulaufs, wie sie beispielsweise bei intervallweisem oder gestuftem bzw. über eine vorgegebene Rampe sich ändernden Zulauf realisiert werden.

Je nach Trennproblem und abhängig von der Lage der Zulaufstelle kann die Kolonne entweder als Abtriebskolonne d.h. alle theoretischen Trennstufen liegen unterhalb der Zulaufstelle (n_{A} = n), Verstärkerkolonne, d.h. alle theoretischen Trennstufen liegen oberhalb der Zulaufstelle (n_{A} = 0), oder als Kolonne mit Abtriebsteil und Verstärkerteil gefahren werden, d.h. n_{A} theoretischen Trennstufen liegen unterhalb, n-n_{A} oberhalb der Zulaufstelle, wobei n die Gesamtzahl der theoretische Trennstufen der Kolonne ist.

Bevorzugt wird die Zulaufstelle so gewählt, dass die Kolonne als Abtriebskolonne gefahren wird.

Als Kolonnen können die dem Fachmann bekannten Kolonnen eingesetzt werden. Beispielsweise sind das Füllkörper oder geordnete Packungen enthaltende Packungskolonnen oder Bodenkolonnen. Ist die Entnahme von Seitenstromfraktionen vorgesehen, kann die Kolonne auch als Trennwandkolonne oder thermisch gekoppelte Kolonne ausgestaltet sein.

Ferner ist es auch möglich, die Kolonne sowohl mit Böden als auch mit Packungselementen auszustatten.

Zur Minimierung von Hold-Up und Druckverlust zur schonenden thermischen Trennung eignen sich bevorzugt Füllkörper oder strukturierte Packungen enthaltende Packungskolonnen sowie entsprechende Bodenkonstruktionen in Bodenkolonnen.

Die für das erfindungsgemäße Verfahren einsetzbaren Böden, Füllkörper und Packungen sind zum Beispiel in Henry Kister, Distillation Design, McGrawHill, 1992 und in K. Sattler, Thermische Trenntechnik, Verlag VCH, 2001 beschrieben.

Die Anzahl der theoretischen Trennstufen in der Kolonne kann beispielsweise 2 bis 150, bevorzugt 2 bis 50, besonders bevorzugt 2 bis 20 und ganz besonders bevorzugt 2 bis 10 betragen.

Die leichtersiedende Nebenfraktion wird üblicherweise dampfförmig über den Kolonnenkopf abgezogen, gegebenenfalls kondensiert und als Kondensat abgezogen.

In einer bevorzugten Ausführungsform des erfindungsgemäßen Verfahrens wird die leichtersiedende Nebenfraktion dampfförmig über den Kolonnenkopf abgezogen, kondensiert und das Kondensat zumindest teilweise wieder der Kolonne zugeführt.

Als Dünnschichtverdampfer können alle bekannten Typen von Dünnschichtverdampfern eingesetzt werden. Bevorzugte Dünnschichtverdampfer sind solche, in denen eine im Innern eines beheizten Zylinders oder Konus mit Hilfe einer drehenden Welle eine dünne Flüssigkeitsschicht erzeugt wird, so dass die über die Wandung eingebrachte Wärme zu einer Verdampfung der Flüssigkeit führt, wobei der Dampf nur einen geringen Weg durch die Flüssigkeit zurücklegen muss.

Vorzugsweise besitzt die Welle starre Abstreifelemente, die einen Spalt geringer Breite (z.B. 1 bis 10 mm) zur beheizten Wand aufweisen oder aber sie ist mit beweglichen Wischerblättern ausgestattet, so dass die über die beheizten Wände des Verdampferkörpers laufende Flüssigkeit in einem dünnen Film gehalten werden kann.

Am Kolonnenfuß fällt üblicherweise eine flüssige Fraktion an, die die mittelsiedende Hauptfraktion und die schwerersiedende Nebenfraktion enthält, die in flüssiger Form dem Dünnschichtverdampfer zugeführt wird und in eine schwerersiedende Nebenfraktion und einen die mittelsiedende Hauptfraktion enthaltenden Dampfstrom getrennt wird.

Die flüssige, schwerersiedende Nebenfraktion wird vorzugsweise aus dem Dünnschichtverdampfer abgezogen.

Der die mittelsiedende Hauptfraktion enthaltende Dampfstrom wird teilweise kondensiert und teilweise der Kolonne zugeführt wird.

Das kann beispielsweise so geschehen, dass der im Dünnschichtverdampfer erzeugte Dampfstrom zunächst im dampfförmigen Zustand geteilt wird, ein Teilstrom in die Kolonne geführt und der andere Teilstrom separat kondensiert und als Produkt abgezogen wird oder der im Dünnschichtverdampfer erzeugte Dampfstrom durch partielle Kondensation geteilt wird und der kondensierte Teil als Produkt abgezogen und der dampfförmige Teilstrom in die Kolonne geführt wird.

Bevorzugt wird der im Dünnschichtverdampfer erzeugte Dampfstrom zunächst im dampfförmigen Zustand geteilt und ein Teilstrom in die Kolonne geführt und der andere Teilstrom separat kondensiert.

Die Kondensation kann beispielsweise in einem von der Kolonne getrennten Wärmetauscher erfolgen. Der Wärmetauscher kann aber auch in die Leitung von Dünnschichtverdampfer zur Kolonne oder in den unteren Kolonnenbereich eingebaut werden, wobei es besonders vorteilhaft ist, den Flüssigkeitsstrom aus der Kolonne zum Dünnschichtverdampfer oberhalb des Wärmetauschers zu sammeln und so zu führen, dass eine möglichst geringe Vermischung dieses Flüssigkeitsstromes mit dem Dampfstrom aus dem Dünnschichtverdampfer zur Kolonne oder mit dem hieraus kondensierten Teilstrom stattfinden kann.

Üblicherweise erfolgt die Trennung je nach Trennproblem bei 0,1 bis 1000 hPa, bevorzugt 1 bis 100 hPa.

Gegebenenfalls kann dem zu trennenden Stoffgemisch ein hochsiedendes Fließmittel zugegeben werden. Hochsiedende Fließmittel sind dabei solche Stoffe, die einen Siedepunkt besitzen, der bezogen auf Normaldruck zumindest 10 K höher, bevorzugt zumindest 20 K höher liegt als der Siedepunkt der mittelsiedenden Hauptfraktion.

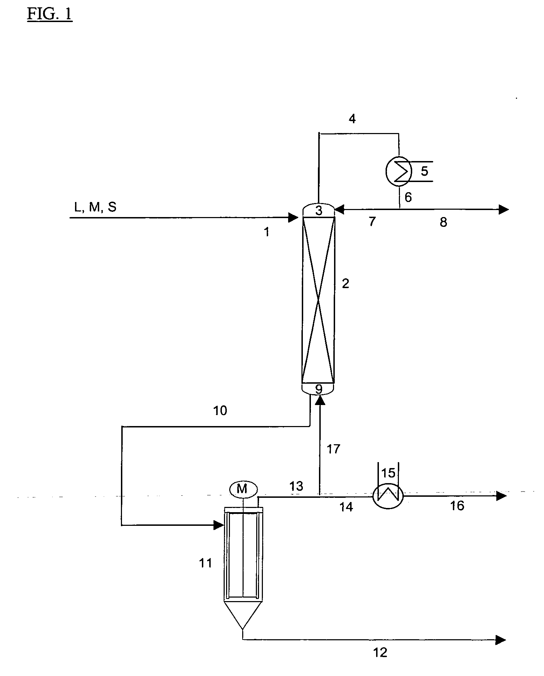

Im Folgenden wird anhand der Figur 1 eine bevorzugte Ausführungsform des erfindungsgemäßen Verfahrens erläutert:

Das zu trennende Flüssigkeitsgemisch enthaltend eine leichtersiedende Nebenfraktion (L), eine mittelsiedende Hauptfraktion (M) und eine schwerersiedende Nebenfraktion (S) wird über eine gegebenenfalls regelbare Zuleitung **1** der Kolonne **2** am oberen Ende zugeführt. Die eine leichtersiedende Nebenfraktion wird am Kolonnenkopf **3** über eine Leitung **4** in den Wärmetauscher **5** geführt und nach Kondensation über die Leitungen **6** und **7** teilweise in die Kolonne **2** zurückgeführt und teilweise über die Leitung **6** und die Ausleitung **8** ausgeschleust.

Am unteren Ende der Kolonne **9** wird ein Flüssigkeitsstrom abgenommen, der die eine mittelsiedende Hauptfraktion und die schwerersiedende Nebenfraktion enthält und über eine Leitung **10** in den Dünnschichtverdampfer **11** eingespeist. Die schwerersiedende Nebenfraktion wird über die Ausleitung **12** als Sumpfprodukt entnommen. Die mittelsiedende Hauptfraktion wird über die Leitung **13** teilweise über eine Leitung **17** als Dampfstrom in die Kolonne **2** geführt und teilweise über die Ausleitung **14** und einen Wärmetauscher **15** als Wertproduktstrom über die Ausleitung **16** entnommen.

Von der Erfmdung sind auch Destillationsvorrichtungen umfasst, die wenigstens bestehen aus
- einer Kolonne, aus deren oberen Teil eine Ausleitung führt und die weiterhin vorzugsweise einen Zulauf besitzt und
- einem von der Kolonne räumlich getrennten Dünnschichtverdampfer, der gegebenenfalls einen Zulauf besitzt und mit dem die Kolonne über eine Zuleitung und im unteren Teil über eine Ableitung verbunden ist, wobei die Zuleitung über einen Wärmetauscher führt oder gegebenenfalls über zumindest eine Leitung mit einem Wärmetauscher verbunden ist und der Wärmetauscher mit einer Ausleitung verbunden ist, und wobei der Dünnschichtverdampfer weiterhin mit einer weiteren Ausleitung verbunden ist.

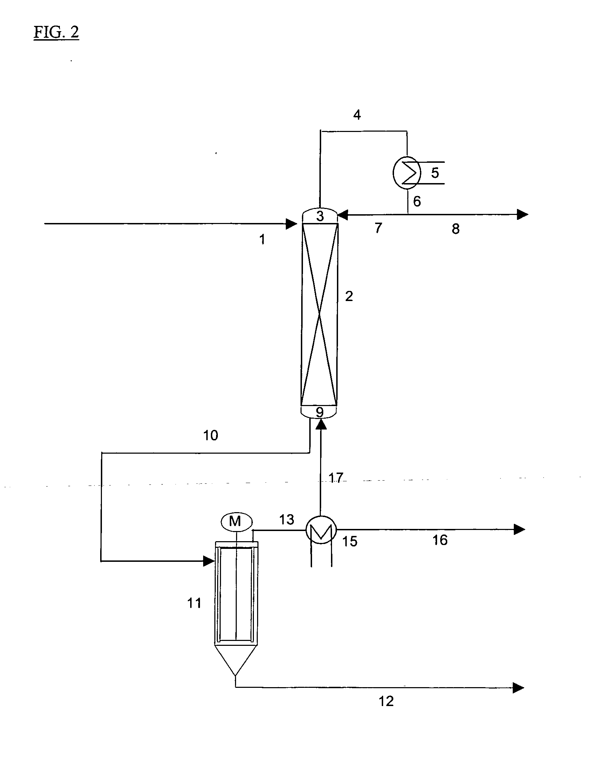

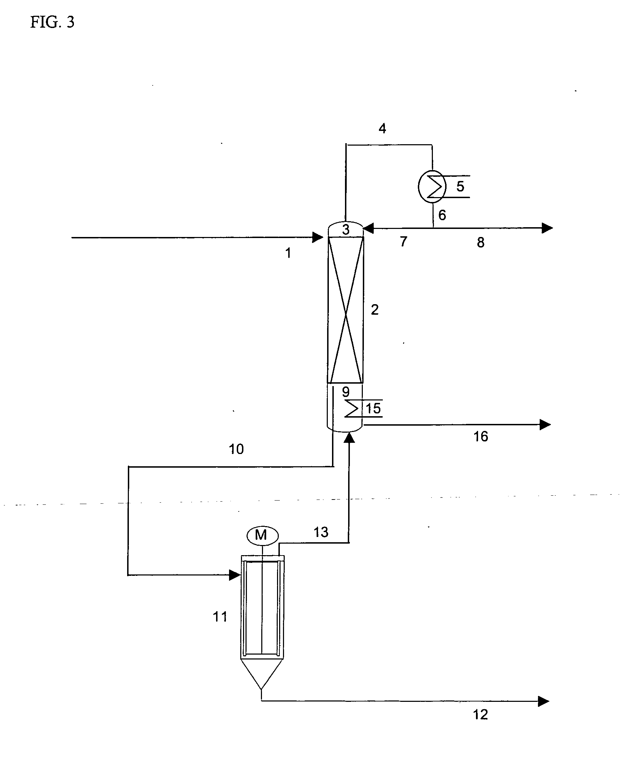

Bevorzugte Destillationsvorrichtungen sind solche, die wenigstens bestehen aus
- einer Kolonne (2), aus deren oberen Teil (3) eine Ableitung (4) über einen Wärmetauscher (5) an eine Leitung (6) führt, die weiterhin über eine Zuleitung (7) mit der Kolonne (2) und weiterhin mit einer Ausleitung (8) verbunden ist, einem gegebenenfalls steuerbaren Zulauf (1), der in die Kolonne (2) mündet und
- einem von der Kolonne räumlich getrennten Dünnschichtverdampfer (11), der über die Ableitung (10) mit dem unteren Teil der Kolonne (9) verbunden ist und mit einer Ausleitung (12) verbunden ist und weiterhin
   - wie in Figur 1 dargestellt, entweder über die Zuleitung (13) mit der Ausleitung (14) und einen Wärmetauscher (15) mit einer Ausleitung (16) verbunden ist und weiterhin über die Zuleitungen (13) und (17) mit der Kolonne (2) verbunden ist oder
   - wie in Figur 2 dargestellt, über die Zuleitung (13), einen Wärmetauscher (15) und über die Zuleitung (17) mit der Kolonne (2) verbunden ist oder
   - wie in Figur 3 dargestellt, über die Zuleitung (13) und einen Wärmetauscher (15) der sich im unteren Teil der Kolonne (2) befindet mit der Kolonne (2) verbunden ist.

Das erfindungsgemäße Verfahren ist insbesondere geeignet für die Zerlegung von Stoffgemischen in mindestens drei Fraktionen. Beispiele für Stoffgemische sind Reaktionsgemische oder Lösungsmittelgemische.

Besonders bevorzugte Stoffgemische sind solche, die Menthol, Menthylchlorid, Menthylpropylenglykolcarbonat und Dimenthylpropandioldicarbonat enthalten.

Nach der Erfmdungsgemäßen Zerlegung erhält man Stoffgemische, die ohne weitere Behandlung insbesondere als Lebensmittelzusätze geeignet sind. Daher sind von der Erfmdung auch Gemische umfasst, die 98,00 Gew.-% bis 99,99 Gew.-% 2-Hydroxypropyl-(2-Isopropyl-5-methylcyclohexyl)-carbonat und 0,001 Gew.-% bis 2,00 Gew.-% 2-Isopropyl-5-methylcyclohexyl-(2-({[(2-isopropyl-5-methylcyclo-hexyl)oxy]carbonyl}oxy)-1-methylethyl)-carbonat enthalten. Bevorzugte Gemische enthalten weiterhin 0,00001 Gew.-% bis 0,01 Gew.-% Menthylchlorid.

Die Zerlegung von solchen Stoffgemischen wird bevorzugt bei 0,1 bis 100, besonders bevorzugt bei 1 bis 50 hPa und ganz besonders bevorzugt bei 1 bis 10 hPa durchgeführt.

Die vorliegende Erfindung zeichnet sich dadurch aus, dass die destillative Zerlegung von Stoffgemischen unter apparativ geringem Aufwand durchgeführt werden kann. Weiterhin können insbesondere Stoffgemische mit thermisch empfindlichen Fraktionen bei sehr guten Raum-Zeit-Ausbeuten schonend in mindestens drei Fraktionen getrennt werden.

### Beispiele

### Beispiel 1

Ein Gemisch aus 5 Gew.-% Wasser (leichtersiedende Nebenfraktion), 90 Gew.-% 1,2-Ethandiol (mittelsiedende Hauptfraktion) und 5 Gew.-% Tetraethylenglykol (schwerersiedende Nebenfraktion) wurde in einer Destillationsvorrichtung in drei Fraktionen zerlegt. Kernstück der Apparatur war eine Kolonne mit einem Durchmesser von 50 mm und einer Packungshöhe von 600 mm. Die Destillationsvorrichtung war ferner mit einem Kopfkondensator und mit einem Dünnschichtverdampfer mit einer Fläche von 0,13 m² ausgestattet. Am unteren Ende der Kolonne war ein zweiter Kondensator (Zwischenkondensator) in die Kolonne integriert, und zwar so, dass der aus dem Dünnschichtverdampfer in die Kolonne strömende Dampf dort teilweise kondensiert wurde und der kondensierte Anteil als Seitenabzug entnommen wurde. Die Flüssigkeit der Kolonne wurde oberhalb des Zwischenkondensators gesammelt und an dem Kondensationsbereich vorbeigeführt, so dass keine Vermischung der Flüssigkeit aus der Kolonne mit dem Kondensat stattfand.

Es wurde ein Zulaufmengenstrom von 1 kg/h eingestellt, wobei der Zulauf zunächst vorgewärmt und dann am oberen Ende der Kolonne zugeführt wurde. Die destillative Trennung erfolgte bei einem Rücklaufverhältnis von 10. Der Kopfdruck der Anlage betrug 100 mbar. Bei diesem Druck stellte sich eine Kopftemperatur von ca. 52°C und eine Sumpftemperatur von ca. 145°C ein. Das Kühlmedium im Zwischenkondensator wurde auf 90°C eingestellt. Mit dieser Fahrweise wurde ein Destillat mit einem Wasseranteil > 95 Gew.-% und ein Sumpfprodukt bestehend aus ca. 50 Gew.-% Glykol und ca. 50 Gew.-% Tetraethylenglykol erhalten. Als Seitenprodukt wurde als mittelsiedende Hauptfraktion Glykol mit einem Gehalt von 99,5 Gew.-% gewonnen. Dabei wurde eine Destillationsausbeute von ca. 94 % erzielt.

### Beispiel 2

Ein Gemisch aus
Menthol (ca. 1 Gew.-%),
Menthylchlorid (ca. 1 Gew.-%),
2-Hydroxypropyl-(2-Isopropyl-5-methylcyclohexyl)-carbonat (ca. 95 Gew.-%) und 2-Isopropyl-5-methylcyclohexyl-(2-({[(2-isopropyl-5-methylcyclo-hexyl)oxy]carbonyl}oxy)-1-methylethyl)-carbonat (ca. 3 Gew.-%)
wurde in der in Beispiel 1 beschriebenen Destillationsvorrichtung in drei Fraktionen zerlegt.

### 2-Hydroxypropyl-(2-isopropyl-5-methylcyclohexyl)-carbonat bezeichnet die Komponente mit folgender Struktur:

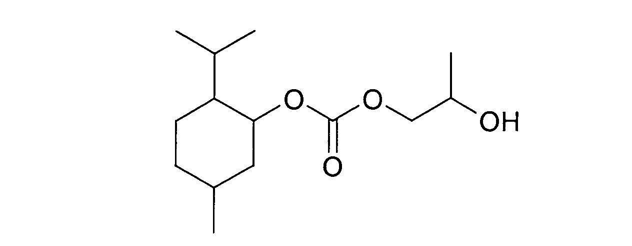

### 2-Isopropyl-5-methylcyclohexyl-(2-({[(2-isopropyl-5-methylcyclohexyl)oxy]car-bonyl}oxy)-1-methylethyl)-carbonat bezeichnet die Komponente mit der Struktur

Das Zulaufgemisch hatte eine Farbzahl nach Hazen von ca. 70.

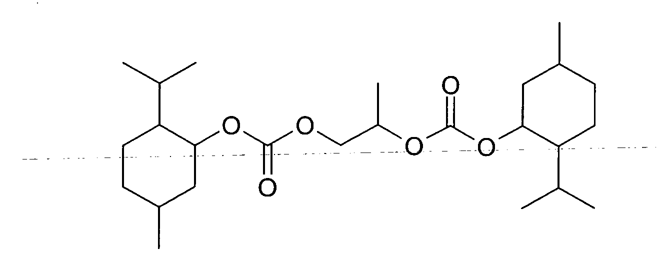

Es wurde ein Zulaufmengenstrom von ca. 650 g/h eingestellt, wobei der Zulauf zunächst vorgewärmt und dann am oberen Ende der Kolonne zugeführt wurde. Die destillative Trennung erfolgte bei einem Rücklaufverhältnis von 10. Der Kopfdruck der Anlage betrug 2,5 mbar. Bei diesem Druck stellte sich am oberen Ende der Kolonne eine Temperatur von ca. 57°C ein. Die Temperatur im Dünnschichtverdampfer betrug ca. 148°C. Das Kühlmedium im Zwischenkondensator wurde auf 140°C eingestellt. Mit dieser Fahrweise wurde als leichtersiedende Nebenfraktion ein Gemisch aus Menthol und Menthylchlorid und als Sumpfprodukt ein Gemisch aus 2-Hydroxypropyl-(2-isopropyl-5-methylcyclohexyl)-carbonat und 2-Isopropyl-5-methylcyclohexyl-(2-({[(2-isopropyl-5-methylcyclo-hexyl)oxy]carbonyl}oxy)-1-methylethyl)-carbonat erhalten. Als mittelsiedende Hauptfraktion wurde 2-Hydroxypropyl-(2-isopropyl-5-methylcyclohexyl)-carbonat mit einer Reinheit von ca. 99,0 Gew.-% und einer Hazen-Farbzahl kleiner 10 abgenommen. Der Anteil an Menthylchlorid in der Hauptfraktion war kleiner als 10 ppm. Der Anteil an 2-Isopropyl-5-methylcyclohexyl-(2-({[(2-isopropyl-5-methylcyclo-hexyl)oxy]carbonyl}oxy)-1-methylethyl)-carbonat in der Hauptfraktion war ca. 0,3 Gew.-%. Dabei wurde für 2-Hydroxypropyl-(2-isopropyl-5-methylcyclohexyl)-carbonat eine Destillationsausbeute von ca. 95 % erreicht.

## Patentansprüche

1. Verfahren zur kontinuierlichen, destillativen Zerlegung von Stoffgemischen bestehend zumindest aus einer mittelsiedenden Hauptfraktion sowie mindestens einer leichtersiedenden und mindestens einer schwerersiedenden Nebenfraktion, **dadurch gekennzeichnet, dass**
• eine Destillationsvorrichtung bestehend zumindest aus einem Dünnschichtverdampfer und einer Kolonne kontinuierlich mit einem Zulauf beaufschlagt wird und
• der Zulauf zumindest in
a) eine leichtersiedende Nebenfraktion und
b) eine Fraktion, die die mittelsiedende Hauptfraktion und die schwerersiedende Nebenfraktion enthält, getrennt wird, und
• die am Kolonnenfuß anfallende Fraktion, die die mittelsiedende Hauptfraktion und die schwerersiedende Nebenfraktion enthält, in flüssiger Form dem Dünnschichtverdampfer zugeführt wird und
• die Fraktion, die die mittelsiedende Hauptfraktion und die schwerersiedende Nebenfraktion enthält, im Dünnschichtverdampfer in
a) eine schwerersiedende Nebenfraktion und
b) einen die mittelsiedende Hauptfraktion enthaltenden Dampfstrom getrennt wird und
• der die mittelsiedende Hauptfraktion enthaltende Dampfstrom teilweise kondensiert und teilweise der Kolonne zugeführt wird.

2. Verfahren zur kontinuierlichen, destillativen Zerlegung von Stoffgemischen bestehend zumindest aus einer mittelsiedenden Hauptfraktion sowie mindestens einer leichtersiedenden und mindestens einer schwerersiedenden Nebenfraktion, **dadurch gekennzeichnet, dass**,
• eine Destillationsvorrichtung, bestehend aus zumindest einem Dünnschichtverdampfer und einer Kolonne, kontinuierlich über den Dünnschichtverdampfer mit einem Zulauf beaufschlagt wird und
• der Zulauf zumindest in
a) einen die leichtersiedende Nebenfraktion und die mittelsiedende Hauptfraktion enthaltenden Dampfstrom und
b) die schwerersiedende Nebenfraktion getrennt wird, und
• aus dem die leichtersiedende Nebenfraktion und die mittelsiedende Hauptfraktion enthaltenden Dampfstrom die mittelsiedende Hauptfraktion zumindest teilweise kondensiert wird und der verbleibende Dampfstrom der Kolonne zugeführt wird und
• der verbleibende Dampfstrom in der Kolonne in einen die leichtersiedende Nebenfraktion enthaltenden Dampfstrom und gegebenenfalls eine am Kolonnenfuß anfallende, die mittelsiedende Hauptfraktion enthaltende Flüssigkeit getrennt wird.

3. Verfahren nach Anspruch 1, **dadurch gekennzeichnet, dass** die Kolonne entweder als Abtriebskolonne, Verstärkerkolonne oder als Kolonne mit Abtriebsteil und Verstärkerteil gefahren wird.

4. Verfahren nach einem der Ansprüche 1 oder 3, **dadurch gekennzeichnet, dass** als Kolonnen Füllkörper oder geordnete Packungen enthaltende Packungskolonnen oder Bodenkolonnen verwendet werden.

5. Verfahren nach einem der Ansprüche 1, 3 oder 4, **dadurch gekennzeichnet, dass** die Anzahl der theoretischen Trennstufen in der Kolonne 2 bis 150 beträgt.

6. Verfahren nach einem der Ansprüche 1 bis 5, **dadurch gekennzeichnet, dass** die leichtersiedende Nebenfraktion dampfförmig über den Kolonnenkopf abgezogen, kondensiert und das Kondensat zumindest teilweise wieder der Kolonne zugeführt wird.

7. Verfahren nach einem der Ansprüche 1 bis 6, **dadurch gekennzeichnet, dass** entweder
- der im Dünnschichtverdampfer erzeugte Dampfstrom zunächst im dampfförmigen Zustand geteilt wird, ein Teilstrom in die Kolonne geführt und der andere Teilstrom separat kondensiert und als Produkt abgezogen wird oder
- der im Dünnschichtverdampfer erzeugte Dampfstrom durch partielle Kondensation geteilt wird und der kondensierte Teil als Produkt abgezogen und der dampfförmige Teilstrom in die Kolonne geführt wird.

8. Verfahren nach einem der Ansprüche 1 bis 7, **dadurch gekennzeichnet, dass** die Zerlegung des Stoffgemisches bei 0,1 bis 1000 hPa durchgeführt wird.

9. Verfahren nach einem der Ansprüche 1 bis 8, **dadurch gekennzeichnet, dass** dem zu trennenden Stoffgemisch ein hochsiedendes Fließmittel zugegeben wird.

10. Verfahren nach einem der Ansprüche 1 bis 9, **dadurch gekennzeichnet, dass** das zu trennende Stoffgemisch enthaltend eine leichtersiedende Nebenfraktion (L), eine mittelsiedende Hauptfraktion (M) und eine schwerersiedende Nebenfraktion (S) über eine gegebenenfalls regelbare Zuleitung **1** der Kolonne **2** am oberen Ende zugeführt wird, die leichtersiedende Nebenfraktion am Kolonnenkopf **3** über eine Leitung **4** in den Wärmetauscher **5** geführt und nach Kondensation über die Leitungen **6** und **7** teilweise in die Kolonne **2** zurückgeführt wird und über die Leitung **6** und die Ausleitung **8** teilweise ausgeschleust wird und am unteren Ende der Kolonne **9** ein Flüssigkeitsstrom abgenommen wird, der die mittelsiedende Hauptfraktion und die schwerersiedende Nebenfraktion enthält und über eine Leitung **10** in den Dünnschichtverdampfer **11** eingespeist wird und die schwerersiedende Nebenfraktion über die Ausleitung **12** als Sumpfprodukt entnommen wird und die mittelsiedende Hauptfraktion über die Zuleitung **13** teilweise über eine Zuleitung **17** als Dampfstrom in die Kolonne **2** geführt und teilweise über die Ausleitung **14** und einen Wärmetauscher **15** als Wertproduktstrom über die Ausleitung **16** entnommen wird.

11. Verfahren nach einem der Ansprüche 1 bis 10, **dadurch gekennzeichnet, dass** das zu trennende Stoffgemisch ein Reaktionsgemisch oder ein Lösungsmittelgemisch ist.

12. Verfahren nach einem der Ansprüche 1 bis 11, **dadurch gekennzeichnet, dass** das zu trennende Stoffgemisch Menthol, Menthylchlorid, 2-Hydroxypropyl-(2-isopropyl-5-methylcyclohexyl)-carbonat und 2-Isopropyl-5-methylcyclohexyl-(2-({[(2-isopropyl-5-methylcyclohexyl)oxy]carbonyl}oxy)-1-methylethyl)-carbonat) enthält.

13. Destillationsvorrichtung, bestehend wenigstens aus
• einer Kolonne, aus deren oberen Teil eine Ausleitung führt und die weiterhin vorzugsweise einen Zulauf besitzt und
• einem von der Kolonne räumlich getrennten Dünnschichtverdampfer, der gegebenenfalls einen Zulauf besitzt und mit dem die Kolonne über eine Zuleitung und im unteren Teil über eine Ableitung verbunden ist, wobei die Zuleitung über einen Wärmetauscher führt oder gegebenenfalls über zumindest eine Leitung mit einem Wärmetauscher verbunden ist und der Wärmetauscher mit einer Ausleitung verbunden ist, und wobei der Dünnschichtverdampfer weiterhin mit einer weiteren Ausleitung verbunden ist.

14. Vorrichtung nach Anspruch 13, **dadurch gekennzeichnet, dass** die Destillationsvorrichtung wenigstens besteht aus
• einer Kolonne (2), aus deren oberen Teil (3) eine Ableitung (4) über einen Wärmetauscher (5) an eine Leitung (6) führt, die weiterhin über eine Zuleitung (7) mit der Kolonne (2) und weiterhin mit einer Ausleitung (8) verbunden ist, einem gegebenenfalls regelbaren Zulauf (1), der in die Kolonne (2) mündet und
• einem von der Kolonne räumlich getrennten Dünnschichtverdampfer (11), der über die Ableitung (10) mit dem unteren Teil der Kolonne (9) verbunden ist und mit einer Ausleitung (12) verbunden ist und weiterhin
- entweder über die Zuleitung (13), die Ausleitung (14) und einen Wärmetauscher (15) mit einer Ausleitung (16) verbunden ist und über die Zuleitungen (13) und (17) mit der Kolonne (2) verbunden ist oder
- über die Zuleitung (13), einen Wärmetauscher (15) und über die Zuleitung (17) mit der Kolonne (2) verbunden ist oder
- über die Zuleitung (13) und einen Wärmetauscher (15) der sich im unteren Teil der Kolonne (2) befindet mit der Kolonne (2) verbunden ist.

15. Gemisch enthaltend 98,00 Gew.-% bis 99,99 Gew.-% 2-Hydroxypropyl-(2-isopropyl-5-methylcyclohexyl)-carbonat und 0,001 Gew.-% bis 2,00 Gew.-% 2-Isopropyl-5-methylcyclohexyl-(2-({[(2-isopropyl-5-methylcyclo-hexyl)oxy]carbonyl}oxy)-1-methylethyl)-carbonat.

16. Gemisch nach Anspruch 15, **dadurch gekennzeichnet dass** es weiterhin 0,00001 Gew.-% bis 0,01 Gew.-% Menthylchlorid enthält.
